# FASCICULE DE BREVET EUROPEEN

(11) **EP 3 167 048 B1**
(45) Date de publication et mention de la délivrance du brevet: **12.09.2018**
(21) Numéro de dépôt: 15734678.4
(22) Date de dépôt: 07.07.2015
(51) Int. Cl.: C12N 5/073, C12N 5/0793, C12N 5/09, A61K 49/00, C12N 5/26, G01N 33/574, A01K 67/027

(54) **MODELE ANIMAL POUR L'ETUDE DU NEUROBLASTOME**
TIERMODELL FÜR DIE UNTERSUCHUNG VON NEUROBLASTOMEN
ANIMAL MODEL FOR STUDYING NEUROBLASTOMAS

(30) Priorité: 07.07.2014 FR 1456531
(43) Date de publication de la demande: 17.05.2017
(73) Titulaire: Université Claude Bernard Lyon I, 69625 Villeurbanne Cedex (FR); Centre National de la Recherche Scientifique, 75794 Paris Cedex 16 (FR)
(72) Inventeur: CASTELLANI, Valérie, 69100 Villeurbanne (FR); DELLOYE-BOURGEOIS, Céline, 69740 Genas (FR)
(74) Mandataire: Regimbeau
(86) Numéro de dépôt international: PCT/EP2015/065509
(87) Numéro de publication internationale: WO 2016/005398

(56) Documents cités:
- US-A1- 2013 171 680
- R CARTER ET AL: "Exploitation of chick embryo environments to reprogram MYCN-amplified neuroblastoma cells to a benign phenotype, lacking detectable MYCN expression", ONCOGENESIS, vol. 1, no. 8, 1 août 2012 (2012-08-01), page e24, XP055190739, DOI: 10.1038/oncsis.2012.24
- ENGLER S ET AL: "A novel metastatic animal model reflecting the clinical appearance of human neuroblasoma: growth arrest of orthotopic tumors by natural, cytotoxic human immunoglobulin M antibodies", CANCER RESEARCH, AMERICAN ASSOCIATION FOR CANCER RESEARCH, US, vol. 61, no. 7, 1 avril 2001 (2001-04-01), pages 2968-2973, XP003002142, ISSN: 0008-5472
- KRISTIN H. KAIN ET AL: "The chick embryo as an expanding experimental model for cancer and cardiovascular research", DEVELOPMENTAL DYNAMICS, vol. 243, no. 2, 19 décembre 2013 (2013-12-19), pages 216-228, XP055190207, ISSN: 1058-8388, DOI: 10.1002/dvdy.24093

## Description

### DOMAINE DE L'INVENTION

La présente invention concerne un modèle d'étude animal du neuroblastome, une tumeur solide pédiatrique représentant 10 % des cancers chez l'enfant.

### ART ANTERIEUR

Le neuroblastome (NB) est une maladie tumorale pédiatrique. L'âge moyen de découverte chez l'enfant est entre un et deux ans. Son incidence est d'environ un cas pour 100 000 enfants, et de très rares cas ont été identifiés chez l'adulte. L'évolution de la maladie est très variable, les formes avec tumeur isolée étant de bien meilleur pronostic que les formes avec métastases. La cause de son apparition est inconnue, la grande majorité des cas étant sporadique et non-familiale.

Cette tumeur dérive des neuroblastes des crêtes neurales embryonnaires (NCCs) et se développe principalement au niveau des ganglions sympathiques et des glandes médullosurrénales. La crête neurale désigne une population de cellules multipotentes générées à partir de la région la plus dorsale du tube neural. Ces cellules migrent dans l'ensemble de l'embryon au cours du développement et donnent naissance à une grande diversité de types cellulaires chez l'adulte. Parmi elles, les neuroblastes se différencient en neurones.

Le micro-environnement embryonnaire au sein duquel émergent les neuroblastomes est spécifique et ne peut pas être étudié sur la majorité des modèles animaux de tumorigenèse, notamment souris et rats, ce qui limite les possibilités de tests précliniques de molécules thérapeutiques candidates.

Des modèles animaux génétiquement modifiés, permettant d'étudier le rôle de l'amplification des oncogènes MYCN ou ALK, fréquemment observées dans les neuroblastomes humains, ont été décrits : il s'agit de souris génétiquement modifiées pour exprimer MYCN (Weiss *et al.,* 1997) ou de zébrafish surexprimant MYCN et/ou ALK (Zhu *et al.,* 2012).

Cependant, les oncogènes MYCN et/ou ALK ne sont pas surexprimés dans tous les types de neuroblastomes observés chez les patients.

A ce jour, il n'existe pas de modèle animal de toutes les formes de neuroblastome, dans lequel des tumeurs se développeraient au sein d'un organisme animal selon la même configuration que dans l'organisme humain, à savoir au sein des ganglions sympathiques et des glandes médullosurrénales. Un tel modèle est cependant nécessaire pour approfondir la connaissance de la maladie, ainsi que pour tester de nouvelles molécules thérapeutiques *in vivo.*

### RESUME DE L'INVENTION

La présente invention est relative à un embryon de gallinacé, préférentiellement de poulet ou de caille, dans lequel ont été greffées, au niveau des crêtes neurales, des cellules humaines de neuroblastome.

Cette greffe est réalisée dans des conditions appropriées permettant à ces cellules de neuroblastome de former des tumeurs au sein des ganglions sympathiques et/ou des glandes médullosurrénales, environ 48h après la greffe. La rapidité de préparation de ce modèle animal est un grand avantage de l'invention.

Cet embryon est un modèle d'étude animal de la pathologie pédiatrique humaine qu'est le neuroblastome. Ce modèle d'étude présente l'avantage de reproduire des tumeurs dans un environnement proche de celui d'origine, à savoir un environnement embryonnaire. Dans ce modèle, les cellules humaines de neuroblastome greffées migrent dans l'embryon animal de la même manière que dans la pathologie humaine, et forment des tumeurs aux sites habituellement colonisés par les neuroblastes : ganglions sympathiques et glandes médullosurrénales.

La présente invention est également relative à un procédé de préparation d'un embryon de gallinacé comprenant les étapes suivantes :
- greffe de cellules humaines de neuroblastome au niveau des crêtes neurales d'un embryon de gallinacé, et
- incubation de l'embryon greffé pendant au moins 24 heures.

La présente invention concerne également un procédé de suivi d'un patient atteint d'un neuroblastome, comprenant :
a) la préparation d'un premier embryon selon le procédé ci-dessus, avec des cellules de neuroblastome issues dudit patient à un instant T1, et l'appréciation de la tumorigenèse des tumeurs se développant dans ce premier embryon,
b) la préparation d'un second embryon selon le procédé ci-dessus, avec des cellules de neuroblastome issues dudit patient à un instant T2, et l'appréciation de la tumorigenèse des tumeurs se développant dans ce second embryon,
c) la comparaison entre la tumorigenèse des tumeurs se développant dans le premier embryon et dans le second embryon.

Ce modèle permet de déterminer les caractéristiques des tumeurs issues des patients, notamment en termes de croissance et de capacité de dissémination. Ces cellules tumorales, qui peuvent notamment être prélevées chez un patient, puis greffées dans un embryon de poulet ou de caille, peuvent être utilisées en particulier pour tester l'efficacité de différentes thérapies *in vivo.* Ceci permet d'administrer au patient des molécules thérapeutiques testées au préalable sur des cellules de sa propre tumeur, greffées dans un embryon de gallinacé, ce qui permet une approche thérapeutique hautement personnalisée.

Ainsi, l'invention concerne aussi un procédé de criblage de molécules thérapeutiques destinées au traitement d'une tumeur neuroblastique *in vivo*, consistant en les étapes suivantes :
a) préparation d'embryons selon le procédé décrit ci-dessus ;
b) administration à ces embryons d'une molécule thérapeutique candidate;
c) appréciation de la tumorigenèse des tumeurs présentes dans les embryons après au moins 24 heures d'administration de ladite molécule.

### LEGENDES DES FIGURES

**Figure 1****.** Représentation des premiers stades de développement de l'embryon de poulet.
**Figure 2****.** Tableau de correspondance des premiers stades de développement des embryons de caille (*Japanese quail*) et de poulet (*chick*).
**Figure 3****.** Représentation schématique du procédé de préparation d'un embryon de gallinacé (Greffe) et de la migration des cellules greffées (Analyse à 48h).

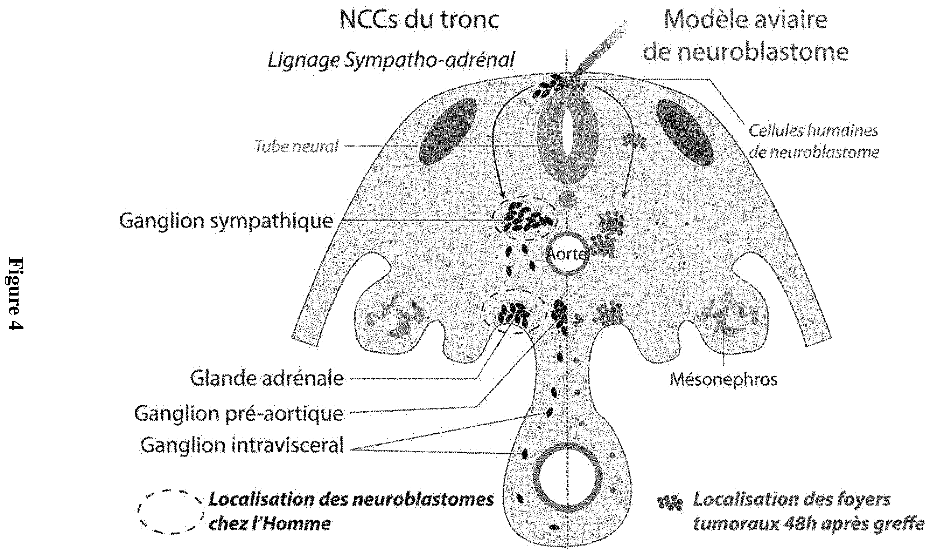

**Figure 4****.** Localisation des foyers tumoraux 48h après la greffe de cellules humaines de neuroblastome (Cercles en pointillés).

### DESCRIPTION DETAILLEE DE L'INVENTION

L'embryon de gallinacé, notamment de poulet ou de caille, est un modèle intéressant pour réaliser des expériences *in vivo,* en particulier pour l'étude du développement embryonnaire et pour des expériences de xéno-transplantation. Il est en effet peu onéreux, très accessible et facile à manipuler. C'est un modèle de choix pour l'étude de la prolifération, de la différentiation et de la migration cellulaire.

En particulier, des cellules marquées, telles que des cellules transformées avec un gène codant pour une protéine fluorescente, ont été utilisées pour visualiser la migration de cellules au cours du développement. Les chimères de caille et de poulet mises au point par N. Le Douarin ont été largement utilisés pour l'étude de la migration des cellules au cours de l'embryogenèse.

Plus récemment, des xénogreffes de cellules humaines dans l'embryon de poulet ont permis l'étude du devenir de ces cellules humaines au cours du développement dudit embryon.

La demande de brevet US 2013/0171680 décrit la xenogreffe de cellules hématopoïétiques humaines malignes dans un embryon de poulet, ces cellules formant des tumeurs au sein de l'embryon. Ce modèle permet de tester de nouvelles molécules thérapeutiques, pour déterminer leur effet au cours du temps sur ces cellules tumorales introduites.

Carter et al. (Oncogenesis, 2012) ont injecté des cellules humaines de neuroblastome issues de tumeurs à mauvais prognostic, surexprimant l'oncogène MYCN, dans les vaisseaux sanguins d'un embryon de poulet (stade 3 et 6 jours). Ces cellules de neuroblastome injectées dans la circulation sanguine se dirigent vers les ganglions sympathiques, mais non vers la glande surrénale. Au contact du micro-environnement embryonnaire, les cellules se re-programment en un phénotype plus bénin, notamment lorsqu'elles se fixent dans les tissus neuronaux. Ce procédé ne permet donc pas de reproduire les tumeurs spécifiquement localisées, caractéristiques de la maladie humaine, chez l'embryon de poulet.

La présente invention est relative à un embryon de gallinacé, préférentiellement de poulet ou de caille, dans lequel ont été greffées, au niveau des crêtes neurales, des cellules humaines de neuroblastome, dans des conditions permettant à ces cellules de neuroblastome de former des tumeurs aux mêmes sites que dans la maladie humaine, au niveau des ganglions sympathiques et des glandes médullosurrénales.

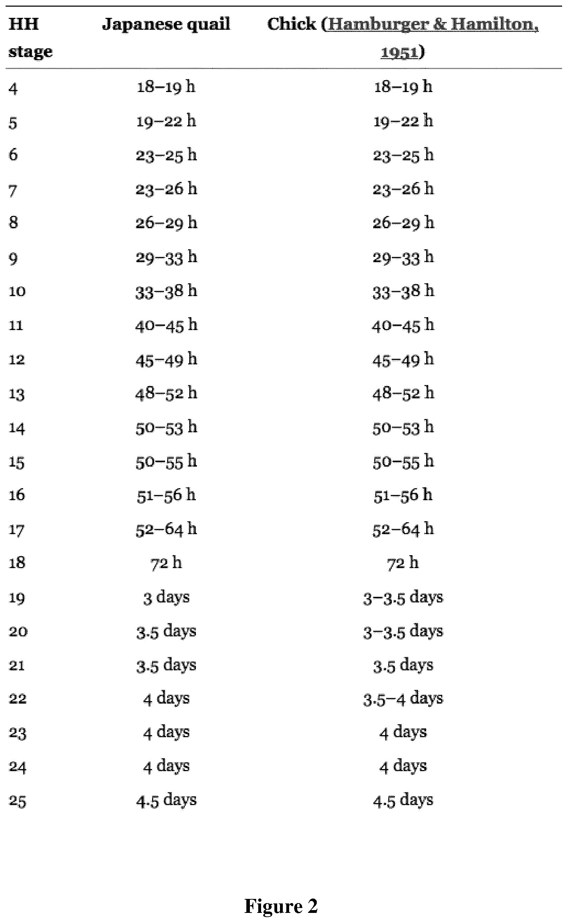

Les termes suivants sont définis pour une meilleure compréhension de l'invention :
Le terme « gallinacé » désigne un oiseau de l'ordre des galliformes (ou gallinacés) qui comprend les poulets, cailles, dindes, faisans, paons, pintades, et d'autres animaux de basse-cour. Préférentiellement, l'embryon sera issu d'un poulet (*gallus gallus*) ou d'une caille (*Coturnix japonica*), deux espèces fréquemment utilisées en laboratoire.
Le terme « embryon de gallinacé » désigne un oeuf de gallinacé fécondé et dans lequel un embryon se développe normalement, dans les conditions adéquates, notamment en étant placé dans un incubateur chauffé à une température comprise entre 37 °C et 39 °C. Plusieurs stades de développement ont été répertoriés et sont indiqués dans la figure 2, colonne de gauche pour l'embryon de caille, colonne de droite pour l'embryon de poulet.
Les termes « embryon », « embryon de gallinacé » et « embryon greffé » sont utilisés indifféremment dans la présente demande, et désignent au sens de l'invention un « embryon chimérique » c'est à dire un embryon possédant des cellules issues d'au moins deux individus différents. Des embryons chimériques caille/poulet ont été abondamment utilisés pour l'étude du développement embryonnaire de ces espèces. Dans ces expériences, des fragments entiers d'un embryon étaient remplacés par les fragments équivalents provenant d'un second embryon, ceci permettant de déterminer le devenir des cellules greffées, distinguables des cellules de l'embryon receveur.

Dans le cadre de la présente invention, l'embryon « receveur » ou « récepteur » est un embryon de gallinacé qui comprend toutes ses propres cellules, et qui comprend de plus des cellules humaines de neuroblastome, qui ont été greffées et deviennent partie intégrante de l'embryon, suite à leur acceptation comme greffon au sein de l'embryon receveur.

Il est entendu que cet embryon chimérique n'est pas, au sens propre, une « chimère », mais désigne un modèle d'étude animal où l'embryon n'est pas destiné à se développer suffisamment de manière à créer un organisme adulte, mais sert uniquement de support aux cellules humaines pendant un court laps de temps. En tout état de cause, il est entendu que cet embryon de gallinacé ne donnera pas naissance à un organisme vivant chimérique, mais sera détruit dès que l'étude du devenir des cellules humaines greffées aura été complétée.

Le terme « greffe » ou « transplantation » désigne l'introduction de cellules exogènes dans un organisme récepteur, à un site précis de l'organisme récepteur, et notamment au sein de tissus spécifiques et non par injection dans la circulation sanguine. La présente demande est en particulier relative à des « xénogreffes » car les cellules introduites sont issues d'un organisme issu d'une espèce différente de l'organisme receveur.

Au sens de l'invention, il est entendu que ces cellules d'origine externe ne sont pas des cellules pluripotentes, et qu'en particulier ces cellules n'ont pas un potentiel de 'cellules souches'.

Le terme « crêtes neurales » désigne, chez l'embryon des vertébrés, une population de cellules transitoires et multipotentes générées à partir de la région la plus dorsale du tube neural. Ces cellules migrent dans l'ensemble de l'embryon au cours du développement et donnent naissance à une grande diversité de types cellulaires chez l'adulte. La crête neurale est notamment à l'origine de toutes les cellules gliales et de la majeure partie des neurones du système nerveux périphérique.

Au sein des crêtes neurales embryonnaires, les neuroblastes sont les cellules progénitrices qui donnent naissance au système nerveux sympathique. Le neuroblastome est une tumeur maligne dérivée des neuroblastes. Elle peut, de ce fait, être retrouvée sur toute la longueur de la colonne vertébrale et dans la partie interne de la glande surrénale. Elle est identifiée en particulier dans les ganglions sympathiques, qui font partie du système nerveux sympathique, et dans la médullosurrénale qui fait partie des glandes surrénales. De manière intéressante, les glandes surrénales existent à la fois chez les mammifères et les oiseaux.

Le terme « cellules humaines de neuroblastome » désigne des cellules tumorales isolées à partir de tumeurs issues du dérèglement de neuroblastes, chez des enfants humains, ou bien des lignées cellulaires immortalisées à partir de ces cellules tumorales.

L'invention est relative à un embryon composé d'un embryon de gallinacé, notamment de poulet ou de caille, sur lequel ont été greffées, au niveau des crêtes neurales, des cellules humaines de neuroblastome, dans des conditions appropriées permettant auxdites cellules de former des tumeurs au sein des ganglions sympathiques et/ou des glandes médullosurrénales.

Ces « conditions appropriées » permettent la reproduction, au sein d'un modèle animal, de la maladie humaine et notamment la formation de tumeurs aux sites spécifiques (ganglions sympathiques et/ou glandes médullosurrénales) de formation de tumeurs observés chez les patients atteints de neuroblastome.

Ces « conditions appropriées » sont basées essentiellement sur le site de la greffe, le stade de développement de l'embryon de gallinacé, et la quantité des cellules humaines greffées.

Les cellules humaines de neuroblastome sont greffées au niveau des crêtes neurales de l'embryon de gallinacé. Selon un aspect préféré de l'invention, les cellules humaines de neuroblastome sont greffées entre les somites 7 à 28 du tube neural de l'embryon, de préférence entre les somites 12 à 24, de manière préférée entre les somites 18 à 24 et plus préférentiellement entre les somites 20 et 21 de l'embryon de gallinacé. En particulier, l'incision permettant la greffe des cellules est réalisée au niveau du toit du tube neural, face aux somites 20 et 21.

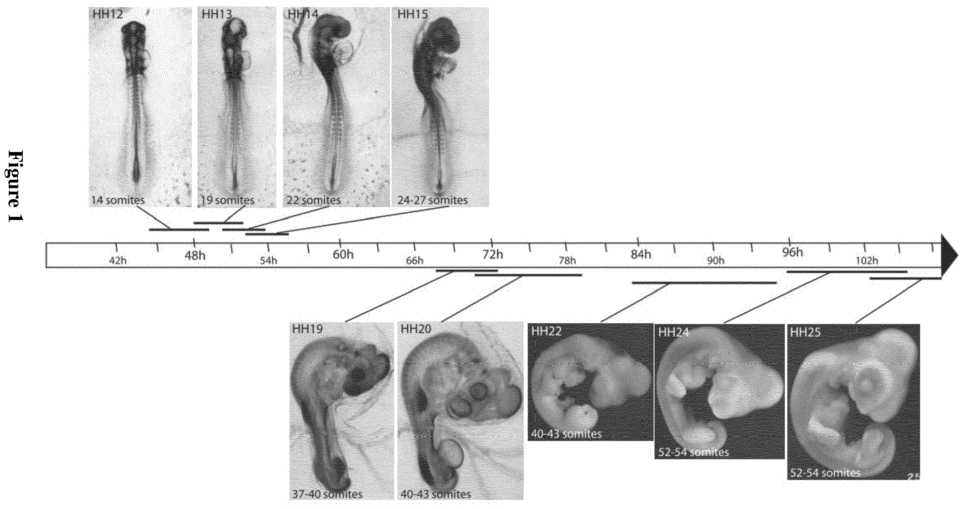

Le tube neural comprend le système nerveux primitif des embryons. Les somites désignent les structures embryonnaires situées de part et d'autre du tube neural et de la chorde, et sont composées d'unités répétées le long de l'axe antéro-postérieur de l'embryon. Au stade de développement compris entre 48 et 55 heures post-fécondation, l'embryon de gallinacé comprend 19 à 28 somites. Une représentation de l'embryon de poulet à différents stades de développement, comprenant de 14 à 54 somites, est présentée en figure 1.

La greffe des cellules humaines de neuroblastome dans l'embryon de gallinacé s'effectue selon les méthodes bien connues de l'homme du métier. L'embryon de gallinacé est en effet facilement accessible, après avoir réalisé une petite ouverture dans la coquille de l'oeuf. En particulier, la greffe des cellules humaines est réalisée à l'aide d'un micro-injecteur sous pression (Picopump PV830, World Precision Instruments). D'autres techniques de transplantation de cellules au sein de l'embryon de gallinacé ont été décrites dans l'art antérieur, par exemple par Kulesa et al. (PNAS, 2006).

Selon un mode de réalisation préféré de l'invention, les cellules humaines de neuroblastome sont greffées en une quantité allant d'environ 15000 cellules à environ 75000 cellules par greffe. En particulier la greffe comprendra environ 15000 cellules, environ 20000, environ 25000, environ 30000, environ 35000, environ 40000, environ 45000, environ 50000, environ 55000, environ 60000, environ 65000, environ 70000, ou encore environ 75000 cellules humaines de neuroblastome.

La méthode permettant de comptabiliser les cellules est bien connue de l'homme du métier. En particulier, le nombre de cellules greffées avec le micro-injecteur est déterminé au préalable de la greffe en comptabilisant à l'aide d'une cellule de comptage de Malassez le nombre de cellules éjectées du capillaire, pendant un temps et à une pression donnée.

Selon un mode préférentiel de réalisation, au moment de la greffe, l'embryon de gallinacé, notamment de poulet ou de caille, est à un stade de développement compris entre 48 et 55 heures post-fécondation, et de préférence entre 50 et 53 heures.

Il est entendu que le développement de l'embryon ne commence que lorsque l'embryon est incubé dans de bonnes conditions, notamment à une température comprise entre 37 C et 39 C. Ainsi, un stade de développement « entre 48 et 55 heures » signifie que l'oeuf a été maintenu cette durée dans les conditions optimales pour son développement. Un oeuf fertilisé peut être maintenu à 14 C avant d'être placé dans des conditions optimales pour son développement ; cette période d'attente à 14 C n'est pas à prendre en compte dans la durée indiquée ci-dessus.

A ce stade de développement, l'embryon de poulet ou de caille comprend entre 19 et 28 somites. Le stade de développement de l'embryon de poulet est déterminé selon les critères définis par Hamburger et Hamilton (1951, J Morphol.), bien connus de l'homme du métier. La figure 2 donne les équivalences entre les stades de développement des embryons de poulet et de caille.

Suite à la greffe, l'embryon de gallinacé est incubé pendant au moins 24 heures selon les techniques standard, dans un incubateur saturé en humidité, à une température comprise entre 37 °C et 39 °C, de manière préférée à 38,5 °C.

Dès 24 heures d'incubation, les premières tumeurs sont observées au sein des glandes médullosurrénales et des ganglions sympathiques de l'embryon de gallinacé.

Selon un aspect particulier de l'invention, l'embryon est incubé après la greffe de cellules humaines de neuroblastome pendant au moins 36 heures, au moins 48 heures, au moins 60 heures, au moins 72 heures, au moins 4 jours, au moins 5 jours, voire jusqu'à l'éclosion de l'oeuf. Selon un aspect préféré de l'invention, l'embryon est incubé pendant environ 48 à 52 heures après la greffe.

Ainsi de distinguer les cellules humaines de neuroblastome au sein de l'embryon de gallinacé, et notamment de suivre leur dispersion et leur capacité de multiplication, les cellules greffées sont avantageusement marquées.

Un tel marquage peut être réalisé à l'aide de colorants. Les cellules peuvent en particulier être marquées grâce à des colorants vitaux tels que des carbocyanides qui ont une affinité pour les membranes cellulaires, auxquelles ils s'incorporent, conférant aux cellules une fluorescence rouge. Les colorants de type succinimidyl esters de carboxyfluorescéine (CFSE), qui émettent une fluorescence verte lorsque qu'ils réagissent avec les protéines intracellulaires pourront également être utilisés.

Selon un aspect particulier de l'invention, les cellules humaines de neuroblastome greffées expriment une protéine marqueur.

Une protéine marqueur désigne une protéine codée par un gène exogène introduit dans la cellule par les méthodes classiques de génie génétique, l'expression de ce gène étant sous le contrôle d'un promoteur actif dans cette cellule, et cette protéine étant visible, ou étant capable de réagir avec un réactif chimique pour devenir visible. De nombreuses protéines marqueurs sont connues telles que la Green Fluorescent Protein (GFP).

Selon un premier aspect de l'invention, les cellules humaines de neuroblastome sont issues d'au moins une lignée cellulaire immortalisée.

L'homme du métier connait plusieurs lignées cellulaires immortalisées de neuroblastome, et notamment les lignées suivantes, utilisées dans les exemples de la présente demande :
- IGR-N-91 et LA-N-5 : voir pour référence l'ouvrage « Neuroblastoma Cell Lines » de Thiele CJ (1998).
- SH-EP et SH-SY5Y : Cancer Res-1989-Ciccarone-219-25. Ces lignées sont commercialisées par l'ATCC sous les appellations suivantes : SH-SY5Y (ATCC® CRL-2266™) et SH-EP (ATCC® CRL-2269™).
- IMR-32: Cancer Res-1970-Tumilowicz-2110-8. Cette lignée est commercialisée par l'ATCC sous l'appellation suivante : IMR-32 (ATCC® CCL-127™).

Selon un aspect particulier de l'invention, plusieurs lignées cellulaires humaines de neuroblastome peuvent être greffées dans l'embryon. Selon un mode de réalisation particulier, dans l'embryon de gallinacé, les cellules humaines de neuroblastome sont issues d'au moins deux lignées cellulaires immortalisées, en particulier deux lignées exprimant chacune une protéine marqueur différente.

Selon un second aspect de l'invention, les cellules humaines de neuroblastome greffées dans l'embryon sont issues d'une tumeur de patient. Ladite tumeur, après prélèvement par chirurgie, est disséquée de manière à isoler au moins 15 000 cellules de neuroblastome ; ces cellules sont ensuite marquées grâce à un colorant vital tels que des carbocyanides ou du succinimidyl ester de carboxyfluorescéine (CFSE) puis greffées sur un embryon au stade de développement compris entre 48 et 55 heures post-fécondation.

L'invention concerne également un procédé de préparation d'un embryon de gallinacé, notamment de poulet ou de caille, comprenant une greffe de cellules humaines de neuroblastome au niveau des crêtes neurales de l'embryon, dans des conditions appropriées permettant auxdites cellules de former des tumeurs au sein des ganglions sympathiques et/ou des glandes médullosurrénales de l'embryon de gallinacé.

En particulier ce procédé de préparation d'un embryon de gallinacé comprend les étapes suivantes :
- greffe de cellules humaines de neuroblastome au niveau des crêtes neurales d'un embryon de gallinacé, et
- incubation de l'embryon greffé pendant au moins 24 heures.

Selon un aspect préféré du procédé, les cellules humaines de neuroblastome sont greffées entre les somites 7 à 28 du tube neural de l'embryon, de préférence entre les somites 12 à 24 et plus préférentiellement entre les somites 18 à 24.

Préférentiellement, les cellules humaines de neuroblastome sont greffées à raison d'environ 15000 cellules à environ 75000 cellules/greffe.

Selon un aspect préféré du procédé, la greffe est réalisée sur un embryon à un stade de développement compris entre 48 et 55 heures.

L'incubation de l'embryon est réalisée dans des conditions standard, à une température comprise entre 37 °C et 39 °C. Selon un aspect particulier de l'invention, l'embryon est incubé après la greffe de cellules humaines de neuroblastome pendant au moins 36 heures, au moins 48 heures, au moins 60 heures, au moins 72 heures, au moins 4 jours, au moins 5 jours, voire jusqu'à l'éclosion de l'oeuf. Selon un aspect préféré de l'invention, le procédé comprend une étape d'incubation de l'embryon greffé pendant environ 48 à 52 heures.

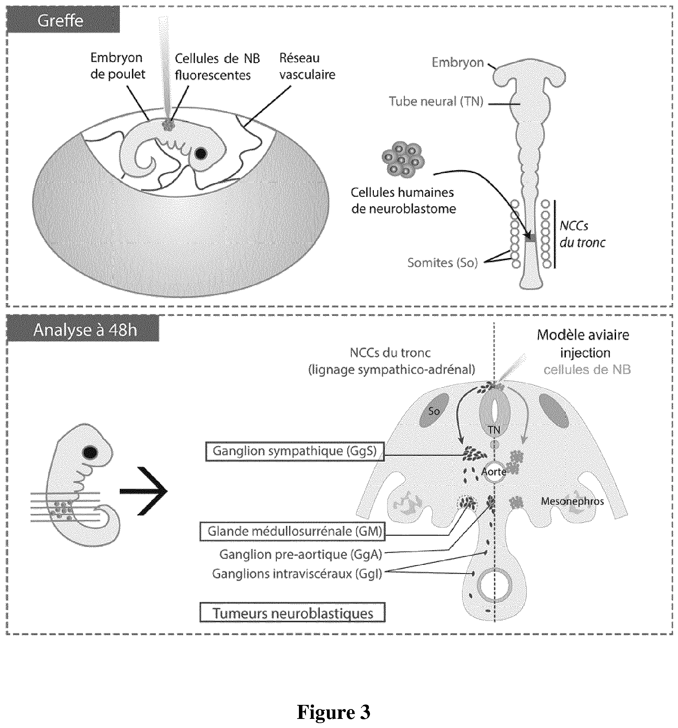

Ce procédé est représenté schématiquement en figure 3.

Selon une première mise en oeuvre de ce procédé, les cellules humaines de neuroblastome sont issues d'au moins une lignée cellulaire immortalisée.

Ainsi, l'invention est relative à un procédé de préparation d'un embryon de gallinacé, comprenant les étapes successives suivantes :
- culture d'au moins une lignée immortalisée de cellules de neuroblastome dans un milieu approprié ;
- greffe de ces cellules, à raison de 15000 cellules à 75000 cellules/greffe, entre les somites 7 à 28, de préférence entre les somites 12 à 24 et plus préférentiellement entre les somites 18 à 24 d'un embryon de gallinacé à un stade de développement compris entre 48 et 55 heures (post-fécondation),
- incubation de l'embryon greffé pendant au moins 24 heures.

Le 'milieu de culture approprié' désigne un milieu de culture adapté à la croissance des cellules, par exemple du milieu DMEM ou du milieu RPMI 1640 (Gibco), supplémentés en antibiotiques et comprenant du sérum de voeu foetal.

De manière préférée, les cellules de la lignée immortalisée expriment une protéine marqueur, ou bien sont marquées à l'aide d'un colorant, comme présenté précédemment.

Les tumeurs au sein des ganglions sympathiques et/ou des glandes médullosurrénales apparaissent dès 24 heures, et sont bien identifiables après 48 heures d'incubation de l'embryon greffé. Ces tumeurs au sein des ganglions sympathiques et/ou des glandes médullosurrénales persistent au moins 96 heures après la greffe.

Selon une seconde mise en oeuvre du procédé, les cellules humaines de neuroblastome sont issues d'une tumeur de patient.

Ainsi, l'invention est relative à un procédé de préparation d'un embryon de gallinacé, comprenant les étapes suivantes :
- obtention de cellules de neuroblastome d'un patient, notamment par prélévement de cellules au sein de sa tumeur ;
- greffe de ces cellules entre les somites 7 à 28, de préférence entre les somites 12 à 24 et plus préférentiellement entre les somites 18 à 24 d'un embryon de gallinacé à un stade de développement compris entre 48 et 55 heures,
- incubation de l'embryon greffé pendant au moins 24 heures.

Les cellules humaines prélevées sur le patient sont dénombrées et greffées à raison d'au moins 15000 cellules / greffe, et de préférence environ 15000 à environ 75000 cellules par greffe.

Ces cellules pourront être détectées au sein de l'embryon receveur, après leur greffe. Cette détection pourra être effectuée selon plusieurs procédés bien connus de l'homme du métier, et notamment :
- par révélation à l'aide d'anticorps reconnaissant un antigène membranaire spécifiquement exprimé par les cellules de neuroblastome. A titre d'exemple, un anticorps reconnaissant le disialoganglioside 2 (GD2), antigène de surface le plus communément exprimé par les cellules neuroblastiques humaines, pourra être utilisé.
- par transformation génétique des cellules de neurobastome, préalablement à leur greffe, avec une cassette d'acide nucléique codant une protéine fluorescente telle que la GFP (Green Fluorescent Protein, fluoresçant en vert), la RFP (Red Fluorescent Protein, fluoresçant en rouge), ou la mCherry (fluoresçant en orange).
- par coloration des cellules de neurobastome, préalablement à leur greffe, avec un colorant cellulaire vital. Des colorants vitaux tels que des carbocyanides qui confèrent aux cellules une fluorescence membranaire rouge ou le succinimidyl ester de carboxyfluorescéine (CFSE), émettant une fluorescence intracellulaire verte, pourront être utilisés.

L'invention se rapporte également à un procédé de suivi d'un patient atteint d'un neuroblastome, comprenant :
a) la préparation d'un premier embryon de gallinacé selon le procédé décrit ci-dessus, avec des cellules de neuroblastome issues dudit patient à un instant T1, et l'appréciation de la tumorigenèse des tumeurs se développant dans ce premier embryon,
b) la préparation d'un second embryon de gallinacé selon le procédé décrit ci-dessus, avec des cellules de neuroblastome issues dudit patient à un instant T2, et l'appréciation de la tumorigenèse des tumeurs se développant dans ce second embryon,
c) la comparaison entre la tumorigenèse des tumeurs se développant dans le premier embryon et dans le second embryon.

Les tumeurs se développant dans les embryons sont issues de la migration des cellules humaines de neuroblastome greffées. Ces tumeurs se développent au sein des ganglions sympathiques et/ou des glandes médullosurrénales. Ces tumeurs sont constituées de cellules humaines de neuroblastome.

« L'appréciation de la tumorigenèse des tumeurs » est effectuée par plusieurs approches complémentaires ; après prélèvement des tumeurs par microdissection, celles-ci sont soumises à différentes analyses :
- des études biochimiques et transcriptomiques, et
- des études *in vitro* via leur remise en culture.

Ces différentes analyses permettent notamment de déterminer les « facteurs de tumorigenèse » suivants :
- Localisation des foyers tumoraux par analyse histologique ;
- Mesure du volume tumoral à partir d'images reconstruites en 3 dimensions ;
- Détermination de l'index prolifératif au sein des foyers tumoraux par détection du marqueur Ki67 ;
- Détermination de l'index de vascularisation des foyers tumoraux par détection de marqueurs d'angiogenèse ;
- Détermination de l'index de mort cellulaire au sein des foyers tumoraux par détection des événements de mort cellulaire (fragmentation de l'ADN, nécrose, relargage du cytochrome c, activation des protéases pro-apoptotiques)
- Analyse du transcriptome et du protéome des tumeurs extraites *in situ* ;
- Etude du comportement cellulaire après remise en culture.

L'analyse combinée de tous ces facteurs, bien connus de l'homme du métier, permet de déterminer un 'indice de tumorigenèse' qui permet de quantifier la gravité et l'agressivité de la tumeur. En effet, l'ensemble de ces paramètres permet d'évaluer l'état de différenciation des cellules neuroblastiques ainsi que leur capacité à proliférer et à se disséminer dans l'organisme. Ces paramètres font partie intégrante de la classification anatomo-pathologique des neuroblastomes sur lesquels s'appuient les cliniciens pour orienter la prise en charge thérapeutique.

Ainsi, il est possible de distinguer :
- des tumeurs se développant suite à la greffe, dans un embryon de gallinacé, de cellules de neuroblastome issues d'un patient à un instant T1, et
- des tumeurs se développant suite à la greffe, dans un embryon de gallinacé, de cellules de neuroblastome issues d'un patient à un instant T2.

Un tel procédé permet de suivre de manière *ex vivo* le développement de la maladie d'un patient, et notamment l'indice de tumorigenèse de ses cellules tumorales à un instant T0 (par exemple, avant le début d'un traitement) et à un instant T1, T2, T3 (par exemple, quelques mois après le début du traitement du patient).

Le procédé peut naturellement être répété le nombre de fois nécessaire pour suivre l'évolution de la maladie chez un patient donné.

L'invention se rapporte enfin à un procédé de criblage de molécules thérapeutiques destinées au traitement d'une tumeur neuroblastique *in vivo,* consistant en les étapes successives suivantes :
a) préparation d'embryons de gallinacé selon l'un des procédés décrit ci-dessus, à partir de cellules immortalisées de neuroblastome ou de cellules de neuroblastome d'un patient ;
b) administration à ces embryons d'une molécule thérapeutique candidate;
c) appréciation de la tumorigenèse des tumeurs présentes dans les embryons après au moins 24 heures d'administration de ladite molécule candidate.

Par « molécule thérapeutique candidate » on entend une molécule chimique ou biologique potentiellement efficace pour traiter le neuroblastome.

L'appréciation de la tumorigenèse des tumeurs est effectuée par les approches décrites ci-dessus, après prélèvement des tumeurs par microdissection. La comparaison de la tumorigenèse des tumeurs au temps T0 et des tumeurs après au moins 24 heures, et en particulier après 1 (T1), 2 (T2) ou 3 (T3) jours d'administration de la molécule testée, permet de déterminer l'effet de la molécule thérapeutique administrée. Naturellement l'administration de cette molécule peut être réalisée pendant différentes durées, notamment pendant au moins 24h, 48 h, 72 h, 96 h, et jusqu'à 5 jours, 6 jours, 7 jours, 8 jours, 9 jours, 10 jours, 11 jours, 12 jours, 13 jours, 14 jours, 15 jours, 16 jours, 17 jours, 18 jours, 19 jours, 20 jours, 21 jours soit jusqu'à l'éclosion de l'oeuf, sous réserve que les tumeurs soient toujours présentes dans l'embryon de gallinacé.

Pour une meilleure compréhension de l'invention, un cas particulier est détaillé ci-dessous :
- Un embryon de gallinacé est préparé avec greffe de cellules tumorales issues d'un patient ;
- Après 48h d'incubation de l'embryon, des tumeurs sont observées au sein de cet embryon ;
- Un premier prélèvement d'une de ces tumeurs est réalisé (T0) et analysé ;
- Une molécule thérapeutique est administré à cet embryon de gallinacé, par les techniques usuelles bien connues de l'homme du métier, notamment par injection dans les vaisseaux sanguins de l'embryon ; l'embryon est maintenu dans des conditions adéquates de température et d'humidité pour son développement ;
- Un second prélèvement de la tumeur est réalisé au bout de 3 jours d'administration de la molécule thérapeutique à l'embryon de gallinacé (T3), et cet échantillon est analysé comme détaillé ci-dessus.

Si l'indice de tumorigenèse de la tumeur prélevée à T0 est élevé (tumeur grave et agressive) ; et si cet indice de tumorigenèse de la tumeur à T3 (après administration de la molécule thérapeutique pendant 3 jours) a diminué, passant de 'élevé' à 'moyen' : l'expérimentateur pourra en conclure que l'effet de la molécule thérapeutique testée est positif.

### EXEMPLES

### MATERIEL ET METHODES

### Lignées cellulaires

Les lignées humaines de neuroblastome ont été obtenues auprès de l'ATCC (SH-EP, IMR-32, SH-SY5Y, LAN-5) ou données par le Dr J. Bénard (IGR-N-91, Institut Gustave Roussy, Villejuif, France). Les lignées SH-EP, IMR-32 et LAN-5 ont été cultivées en milieu RPMI 1640 (Gibco) et les lignées IGR-N-91 et SH-SY5Y en milieu DMEM (Gibco). Chaque milieu a été supplémenté avec 25 Unités/mL de Pénicilline-Streptomycine (Gibco), 2,5µg/mL d'Amphotéricine B (Gibco) et 10 % de Sérum de Veau Foetal (Gibco).

### Embryons de poulet

Les oeufs de poulet (*Gallus gallus*) fertilisés ont été achetés auprès d'un fournisseur local (EARL Morizeau, Dangers, France) et maintenus à 14 °C jusqu'à utilisation. Les oeufs ont été incubés à 38,5 °C pendant 52 heures dans un incubateur au taux d'humidité saturé, de façon à obtenir des embryons au stade de développement HH13 à HH16.

### Greffes de lignées tumorales humaines dans l'embryon de poulet

5x10⁶ cellules tumorales ont été récoltées et resuspendues dans 30 µL de milieu.

Après 52 heures d'incubation à 38,5 °C, une fenêtre a été découpée dans la coquille afin de visualiser et d'accéder à l'embryon. La membrane vitelline a été découpée au niveau du tube neural et une blessure a été réalisée au niveau du toit du tube neural, face aux somites 20 et 21. Les cellules ont été insérées dans un micro-capillaire en verre et déposées à l'aide d'un micro-injecteur sous pression (Picopump PV830, World Precision Instruments). Les cellules ont ainsi été greffées au niveau du toit dorsal du tube neural sur une zone correspondant à 1 à 2 somites. Les oeufs ont ensuite été replacés dans l'incubateur à 38,5 °C pendant 52 heures.

### Coupes d'embryons de poulet

Les embryons ont été récoltés et fixés en paraformaldéhyde 4 %, sur la nuit à 4 °C. Selon le type d'analyse souhaitée, les embryons ont été coupés pour la réalisation de coupes transversales et sagittales. Les coupes ont été maintenues en PBS à 4 °C à l'abri de la lumière jusqu'à utilisation. La localisation des tumeurs a été étudiée par différents immuno-marquages et détection de la fluorescence des cellules tumorales préalablement transduites pour exprimer la GFP ou la RFP (protéines marqueurs).

### Prélèvement des tumeurs in situ et analyse

Les tumeurs sont prélevées par microdissection et soumises à différentes analyses :
- des études biochimiques et transcriptomiques (caractérisation et recherche de marqueurs moléculaires connus ou nouveaux), et
- des études *in vitro* via leur remise en culture.

### Prise d'images et traitement

Les coupes ont été analysées à l'aide d'un microscope confocal (Olympus IX81). L'image intégrale de la coupe a été reconstituée en utilisant le logiciel XuvTools.

La tumorigenèse a été appréciée à l'aide de différentes analyses :
Détermination de la localisation des foyers tumoraux par analyse histologique
Mesure du volume tumoral à partir des images reconstruites en 3 dimensions
Détermination de l'index prolifératif au sein des foyers tumoraux par détection du marqueur Ki67
- Détermination de l'index de vascularisation des foyers tumoraux par détection de marqueurs d'angiogenèse
- Détermination de l'index de mort cellulaire au sein des foyers tumoraux par détection des événements de mort cellulaire (fragmentation de l'ADN, nécrose, relargage du cytochrome c, activation des protéases pro-apoptotiques)
- Analyse du transcriptome et du protéome des tumeurs extraites *in situ*
- Etude du comportement cellulaire après remise en culture

### RESULTATS

Cinq lignées humaines de neuroblastome de stade 4 : IGR-N-91, Lan5, SH-SY5Y, SH-EP, et IMR32, ont été greffées au niveau du toit dorsal de la moelle épinière d'embryons de poulet, où siègent les crêtes neurales sympatho-adrénales. Dans chaque embryon une seule lignée cellulaire a été introduite, à raison d'environ 15000 à 75000 cellules / greffe.

48 heures après la greffe, dans 60 à 95 % des embryons selon la lignée greffée, les cellules fluorescentes ont quitté le site de greffe et ont adopté une trajectoire ventrale, similaire à celle empruntée par les cellules de crêtes neurales endogènes. La formation de masses tumorales a été observée exclusivement au niveau des ganglions sympathiques, des glandes médullosurrénales et/ou entre la moelle épinière et les ganglions dorso-rachidiens. La localisation de ces foyers tumoraux est identique aux sites de développement des neuroblastomes chez l'enfant.

72h et 96h après la greffe, ces masses tumorales se maintiennent, s'amplifient et sont associées à des foyers secondaires principalement détectés au niveau du rétropéritoine, site majeur de développement des métastases neuroblastiques chez l'homme. De façon intéressante, ce comportement semble spécifique des lignées de neuroblastome. En effet, des cellules fluorescentes de mélanome ou de carcinome pulmonaire greffées chez l'embryon de poulet selon un protocole identique ne migrent pas selon un trajet « ventral » et ne s'établissent pas dans les dérivés sympathico-adrénaux.

D'autre part, la possibilité de greffer, au sein du même embryon de poulet, deux lignées de neuroblastome exprimant une protéine fluorescente différente, a été étudiée. Ainsi, la localisation tissulaire des cellules de chacune des lignées peut être observée distinctement, 48h après la greffe. Ceci permet de comparer directement, en évitant tout biais lié à une possible variabilité entre embryon, le comportement de deux lignées de neuroblastome. Par exemple, des cellules ayant été soumises à un traitement au préalable de la greffe peuvent être directement comparées à leurs homologues non traitées. De même, les éventuelles différences comportementales de prélèvements tumoraux de patients avant et après traitements médicamenteux peuvent être aisément mesurées.

### REFERENCES BIBLIOGRAPHIQUES

### BREVETS

US 2013/0171680

### NON BREVETS

Weiss WA, Aldape K, Mohapatra G, Feuerstein BG, Bishop JM. Targeted expression of MYCN causes neuroblastoma in transgenic mice. EMBO J. 1997 Jun 2;16(11):2985-95.
Zhu S, Lee JS, Guo F, Shin J, Perez-Atayde AR, Kutok JL, Rodig SJ, Neuberg DS, Helman D, Feng H, Stewart RA, Wang W, George RE, Kanki JP, Look AT. Activated ALK collaborates with MYCN in neuroblastoma pathogenesis. Cancer Cell. 2012 Mar 20;21(3):362-73.
R Carter, D Mullassery, V See, S Theocharatos, B Pizer, PD Losty, E Jesudason and DJ Moss. Exploitation of chick embryo environments to reprogram MYCN-amplified neuroblastoma cells to a benign phenotype, lacking detectable MYCN expression. Oncogenesis. 2012 Aug 27;1:e24
Kulesa PM, Kasemeier-Kulesa JC, Teddy JM, Margaryan NV, Seftor EA, Seftor RE, Hendrix MJ. Reprogramming metastatic melanoma cells to assume a neural crest cell-like phenotype in an embryonic microenvironment. Proc Natl Acad Sci U S A. 2006 Mar 7;103(10):3752-7.
Hamburger V, Hamilton HL. A series of normal stages in the development of the chick embryo. J Morphol. 1951 Jan;88(1):49-92.
Thiele CJ. Neuroblastoma: In (Ed.) Masters, J. Human Cell Culture. Lancaster, UK: Kluwer Academic Publishers. 1998, Vol 1, p 21-53.
Ciccarone V, Spengler BA, Meyers MB, Biedler JL, Ross RA. Phenotypic diversification in human neuroblastoma cells: expression of distinct neural crest lineages. Cancer Res. 1989 Jan 1;49(1):219-25.
Tumilowicz JJ, Nichols WW, Cholon JJ, Greene AE. Definition of a continuons human cell line derived from neuroblastoma. Cancer Res. 1970 Aug;30(8):2110-8.

## Revendications

1. Embryon de gallinacé dans lequel ont été greffées, au niveau des crêtes neurales, des cellules humaines de neuroblastome.

2. Embryon de gallinacé selon la revendication 1, **caractérisé en ce que** les cellules humaines de neuroblastome sont greffées entre les somites 7 à 28 du tube neural de l'embryon, de préférence entre les somites 12 à 24 et plus préférentiellement entre les somites 18 à 24.

3. Embryon de gallinacé selon l'une des revendications 1 à 2, **caractérisé en ce que** les cellules humaines de neuroblastome sont greffées en une quantité allant d'environ 15000 cellules à environ 75000 cellules par greffe.

4. Embryon de gallinacé selon l'une des revendications 1 à 3, **caractérisé en ce que** la greffe est réalisée sur un embryon à un stade de développement compris entre 48 et 55 heures.

5. Embryon de gallinacé selon l'une des revendications 1 à 4, **caractérisé en ce que** l'embryon est incubé pendant au moins 24 heures après la greffe.

6. Embryon de gallinacé selon l'une des revendications 1 à 5, **caractérisé en ce que** les cellules humaines de neuroblastome expriment une protéine marqueur.

7. Embryon de gallinacé selon l'une des revendications 1 à 6, **caractérisé en ce que** les cellules humaines de neuroblastome sont issues d'au moins une lignée cellulaire immortalisée.

8. Embryon de gallinacé selon la revendication 7, **caractérisé en ce que** les cellules humaines de neuroblastome sont issues de deux lignées cellulaires immortalisées exprimant chacune une protéine marqueur différente.

9. Embryon de gallinacé selon l'une des revendications 1 à 6, **caractérisé en ce que** les cellules humaines de neuroblastome sont issues d'une tumeur de patient.

10. Procédé de préparation d'un embryon de gallinacé comprenant les étapes suivantes :
- greffe de cellules humaines de neuroblastome au niveau des crêtes neurales d'un embryon de gallinacé, et
- incubation de l'embryon greffé pendant au moins 24 heures.

11. Procédé selon la revendication 10, **caractérisé en ce que** les cellules humaines de neuroblastome sont greffées entre les somites 7 à 28 du tube neural de l'embryon, de préférence entre les somites 12 à 24 et plus préférentiellement entre les somites 18 à 24.

12. Procédé selon l'une des revendications 10 ou 11, **caractérisé en ce que** les cellules humaines de neuroblastome sont greffées en une quantité allant d'environ 15000 cellules à environ 75000 cellules par greffe.

13. Procédé selon l'une des revendications 10 à 12, **caractérisé en ce que** la greffe est réalisée sur un embryon à un stade de développement compris entre 48 et 55 heures.

14. Procédé selon l'une des revendications 10 à 13, **caractérisé en ce que** les cellules humaines de neuroblastome sont issues d'au moins une lignée cellulaire immortalisée.

15. Procédé selon l'une des revendications 10 à 13, **caractérisé en ce que** les cellules humaines de neuroblastome sont issues d'une tumeur de patient.

16. Procédé de suivi d'un patient atteint d'un neuroblastome, comprenant :
a) la préparation d'un premier embryon selon le procédé de la revendication 15, avec des cellules de neuroblastome issues dudit patient à un instant T1, et l'appréciation de la tumorigenèse des tumeurs se développant dans ce premier embryon,
b) la préparation d'un second embryon selon le procédé de la revendication 15, avec des cellules de neuroblastome issues dudit patient à un instant T2, et l'appréciation de la tumorigenèse des tumeurs se développant dans ce second embryon,
c) la comparaison entre la tumorigenèse des tumeurs se développant dans le premier embryon et dans le second embryon.

17. Procédé de criblage de molécules thérapeutiques destinées au traitement d'une tumeur neuroblastique, consistant en les étapes suivantes :
a) préparation d'embryons selon le procédé de l'une des revendications 10 à 15 ;
b) administration à ces embryons d'une molécule thérapeutique candidate;
c) appréciation de la tumorigenèse des tumeurs présentes dans les embryons après au moins 24 heures d'administration de ladite molécule candidate.

## Patentansprüche

1. Hühnervogel-Embryo in den, auf der Ebene der Neuralleisten menschliche Neuroblastomzellen verpflanzt wurden.

2. Hühnervogel-Embryo nach Anspruch 1, **dadurch gekennzeichnet, dass** die menschlichen Neuroblastomzellen zwischen den Somiten 7 bis 28 des Neuralrohrs des Embryos verpflanzt sind, vorzugsweise zwischen den Somiten 12 bis 24 und noch bevorzugter zwischen den Somiten 18 bis 24.

3. Hühnervogel-Embryo nach einem der Ansprüche 1 bis 2, **dadurch gekennzeichnet, dass** die menschlichen Neuroblastomzellen in einer Menge verpflanzt sind, die von ungefähr 15000 Zellen bis ungefähr 75000 Zellen pro Verpflanzung reicht.

4. Hühnervogel-Embryo nach einem der Ansprüche 1 bis 3, **dadurch gekennzeichnet, dass** die Verpflanzung auf einem Embryo in einem Entwicklungsstadium im Bereich zwischen 48 und 55 Stunden durchgeführt wird.

5. Hühnervogel-Embryo nach einem der Ansprüche 1 bis 4, **dadurch gekennzeichnet, dass** der Embryo während mindestens 24 Stunden nach der Verpflanzung inkubiert wird.

6. Hühnervogel-Embryo nach einem der Ansprüche 1 bis 5, **dadurch gekennzeichnet, dass** die menschlichen Neuroblastomzellen ein Markerprotein exprimieren.

7. Hühnervogel-Embryo nach einem der Ansprüche 1 bis 6, **dadurch gekennzeichnet, dass** die menschlichen Neuroblastomzellen aus mindestens einer immortalisierten Zelllinie stammen.

8. Hühnervogel-Embryo nach Anspruch 7, **dadurch gekennzeichnet, dass** die menschlichen Neuroblastomzellen aus zwei immortalisierten Zelllinien stammen, die jeweils ein verschiedenes Markerprotein exprimieren.

9. Hühnervogel-Embryo nach einem der Ansprüche 1 bis 6, **dadurch gekennzeichnet, dass** die menschlichen Neuroblastomzellen aus einem Patiententumor stammen.

10. Verfahren zur Herstellung eines Hühnervogel-Embryos, umfassend die folgenden Schritte:
- Verpflanzen von menschlichen Neuroblastomzellen auf der Ebene der Neuralleisten eines Hühnervogel-Embryos, und
- Inkubieren des verpflanzten Embryos während mindestens 24 Stunden.

11. Verfahren nach Anspruch 10, **dadurch gekennzeichnet, dass** die menschlichen Neuroblastomzellen zwischen den Somiten 7 bis 28 des Neuralrohrs des Embryos verpflanzt sind, vorzugsweise zwischen den Somiten 12 bis 24 und noch bevorzugter zwischen den Somiten 18 bis 24.

12. Verfahren nach einem der Ansprüche 10 oder 11, **dadurch gekennzeichnet, dass** die menschlichen Neuroblastomzellen in einer Menge verpflanzt sind, die von ungefähr 15000 Zellen bis ungefähr 75000 Zellen pro Verpflanzung reicht.

13. Verfahren nach einem der Ansprüche 10 bis 12, **dadurch gekennzeichnet, dass** die Verpflanzung auf einem Embryo in einem Entwicklungsstadium im Bereich zwischen 48 und 55 Stunden durchgeführt wird.

14. Verfahren nach einem der Ansprüche 10 bis 13, **dadurch gekennzeichnet, dass** die menschlichen Neuroblastomzellen aus mindestens einer immortalisierten Zelllinie stammen.

15. Verfahren nach einem der Ansprüche 10 bis 13, **dadurch gekennzeichnet, dass** die menschlichen Neuroblastomzellen aus einem Patiententumor stammen.

16. Verfahren zur Pflege eines Patienten, der an einem Neuroblastom leidet, umfassend:
a) Herstellen eines ersten Embryos gemäß dem Verfahren von Anspruch 15 mit Neuroblastomzellen des Patienten zu einem Zeitpunkt T1, und Beurteilen der Tumorgenese der Tumore, die sich in diesem ersten Embryo entwickeln,
b) Herstellen eines zweiten Embryos gemäß dem Verfahren von Anspruch 15 mit Neuroblastomzellen des Patienten zu einem Zeitpunkt T2 und Beurteilen der Tumorgenese der Tumore, die sich in diesem zweiten Embryo entwickeln,
c) Vergleichen zwischen der Tumorgenese der Tumore, die sich im ersten Embryo und im zweiten Embryo entwickeln.

17. Verfahren des Screenens von therapeutischen Molekülen, die für die Behandlung von einem neuroblastischen Tumor vorgesehen sind, bestehend aus den folgenden Schritten:
a) Herstellen von Embryos gemäß dem Verfahren von einem der Ansprüche 10 bis 15;
b) Verabreichen an diese Embryos eines therapeutischen Kandidatenmoleküls;
c) Beurteilen der Tumorgenese der Tumore, die in den Embryos vorhanden sind, nach mindestens 24 Stunden Verabreichung des Kandidatenmoleküls.

## Claims

1. Gallinaceous embryo onto which human neuroblastoma cells have been grafted at neural crests.

2. Gallinaceous embryo according to claim 1, **characterised in that** the human neuroblastoma cells are grafted between somites 7 and 28 of the neural tube of the embryo, preferably between somites 12 and 24 and more preferably between somites 18 and 24.

3. Gallinaceous embryo according to either claim 1 or 2, **characterised in that** the quantity of grafted human neuroblastoma cells is between about 15000 cells and about 75000 cells per graft.

4. Gallinaceous embryo according to one of claims 1 to 3, **characterised in that** the graft is made on an embryo at a development stage between 48 and 55 hours.

5. Gallinaceous embryo according to one of claims 1 to 4, **characterised in that** the embryo is incubated for at least 24 hours after the graft.

6. Gallinaceous embryo according to one of claims 1 to 5, **characterised in that** the human neuroblastoma cells express a marker protein.

7. Gallinaceous embryo according to one of claims 1 to 6, **characterised in that** the human neuroblastoma cells are derived from at least one immortalised cell line.

8. Gallinaceous embryo according to claim 7, **characterised in that** the human neuroblastoma cells are derived from two immortalised cell lines each expressing a different marker protein.

9. Gallinaceous embryo according to one of claims 1 to 6, **characterised in that** the human neuroblastoma cells are derived from a patient tumour.

10. Method of preparing a gallinaceous embryo comprising the following steps:
- grafting human neuroblastoma cells at the neural crests of a gallinaceous embryo, and
- incubating the grafted embryo for at least 24 hours

11. Method according to claim 10, **characterised in that** the human neuroblastoma cells are grafted between somites 7 and 28 of the neural tube of the embryo, preferably between somites 12 and 24 and more preferably between somites 18 and 24.

12. Method according to one of claims 10 or 11, **characterised in that** the quantity of grafted human neuroblastoma cells is between about 15000 cells and about 75000 cells per graft.

13. Method according to one of claims 10 to 12, **characterised in that** the graft is made on an embryo at a development stage of between 48 and 55 hours.

14. Method according to one of claims 10 to 13, **characterised in that** the human neuroblastoma cells are derived from at least one immortalised cell line.

15. Method according to one of claims 10 to 13, **characterised in that** the human neuroblastoma cells are derived from a patient tumour.

16. Method for monitoring a patient suffering from a neuroblastoma, comprising:
a) preparation of a first embryo according to the method described in claim 15, with neuroblastoma cells derived from said patient at time T1, and evaluation of the tumorigenesis of tumours developing in this first embryo,
b) preparation of a second embryo using the method described in claim 15, with neuroblastoma cells derived from said patient at time T2, and evaluation of the tumorigenesis of tumours developing in thus second embryo,
c) comparison between the tumorigenesis of tumours developing in the first embryo and in the second embryo.

17. Method of screening therapeutic molecules for use in the treatment of a neuroblastoma tumour consisting of the following steps.
a) preparation of embryos using the method according to one of claims 10 to 15,
b) administration of a candidate therapeutic molecule to these embryos,
c) assessment of the tumorigenesis of tumours present in the embryos after at least 24 hours of administration of the second candidate molecule.
